(19) Europäisches Patentamt
European Patent Office
Office européen des brevets

(11) Veröffentlichungsnummer: **0 126 877**
**A1**

# (12) EUROPÄISCHE PATENTANMELDUNG

(21) Anmeldenummer: **84103034.9**

(22) Anmeldetag: **20.03.84**

(51) Int. Cl.³: **C 12 P 33/02**
C 07 J 5/00

(30) Priorität: **27.04.83 DE 3315722**

(43) Veröffentlichungstag der Anmeldung:
**05.12.84 Patentblatt 84/49**

(84) Benannte Vertragsstaaten:
**AT BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Manecke, Georg, Prof. Dr.**
**Hittorfstrasse 29**
**D-1000 Berlin 33(DE)**

(72) Erfinder: **Beier, Wilfried, Dipl.-Chem.**
**Reuterplatz 1**
**D-1000 Berlin 44(DE)**

## (54) Verfahren zur Herstellung von 3-Oxo-delta 1,4-steroiden.

(57) Ein Verfahren zur Herstellung von 3-0xo-$\Delta^{1,4}$-steroiden aus in der 1,2-Position gesättigten 3-0xo-$\Delta^4$-steroiden durch Fermentation mit einem Biokatalysator, in welchem Mikroorgnismen der Spezies Arthrobacter simplex oder Bacillus sphaericus in vernetztem, mit einer Mercaptocarbonsäure der allgemeinen Formel I

$$HOOCC_nH_{2n}SH \qquad (I),$$

worin n die Ziffern 1 bis 5 bedeuten, veresterten Polyvinylalkohol immobilisiert sind, wird beansprucht, daß dadurch gekennzeichnet ist, daß man den Biokatalysator einer wässrigen Suspension des mikronisierten 3-0xo-$\Delta^4$-steroids zusetzt, die Reaktionsmischung bei einer Temperatur von 20 bis 40° C bis zur Umsetzung des Steroids inkubiert und danach den Biokatalysator mechanisch von der wässrigen Suspension des gebildeten 3-0xo-$\Delta^{1,4}$-steroids abtrennt.

EP 0 126 877 A1

Die Erfindung betrifft das in den Patentansprüchen gekennzeichnete Verfahren.

Bekanntlich werden Mikroorganismen-Kulturen der Species Arthrobacter simplex (wie z.B. ATCC 6946,13260 und IFO 3530) oder Bacillus sphaericus (wie z.B. ATCC 7054,7055,12488 und 13805) zur $\Delta^1$-Dehydrierung von 3-Keto-$\Delta^4$-steroiden verwendet.

Es ist vorbekannt, daß Mikroorganismen der Species Arthrobacter simplex, die in vernetztem, mit 3-Mercaptopropionsäure veresterten Polyvinylalkohol immobilisiert sind, ebenfalls die Fähigkeit besitzen 3-Keto-$\Delta^4$-steroide zu 3-Keto-$\Delta^{1,4}$-steroiden zu dehydrieren. (Die Angewandte Makromolekulare Chemie 104, 1982, 39-58.) Auch aus Mikroorganismen der Species Bacillus sphaericus lassen sich in gleicher Weise Biokatalysatoren herstellen.

Diese für die Herstellung des Biokatalysators benötigten Mikroorganismen werden in üblicher Weise angezüchtet und nach erfolgter Anzucht abfiltriert oder abzentrifugiert, mit Wasser oder verdünnter Pufferlösung gewaschen und nochmals filtriert oder zentrifugiert. Die so erhaltene feuchte Biomasse kann ohne weitere Aufarbeitung zur Herstellung des Biokatalysators verwendet werden, wobei man etwa die 0,2 bis 15-fache vorzugsweise 3 bis 10-fache Menge feuchte Biomasse bezogen auf den vernetzten mit der Mercaptocarbonsäure veresterten Polyvinylalkohol (Trockenmasse) verwendet. Andererseits kann aber diese Biomasse auch durch Sprühtrocknung getrocknet werden und man setzt dann 0,5 bis 5 mal vorzugsweise 0,1 bis 3 mal so viel Trockenpulver wie Polymerisat (Trockenmasse) ein.

Zur Herstellung des mit der Mercaptocarbonsäure veresterten Polyvinylalkohols verwendet man vorzugsweise Polyvinylalkohol, welcher ein Molekulargewicht von 20 000 bis 200 000 hat.

Die zur Veresterung des Polyvinylalkohols verwendete Mercaptocarbonsäure kann verzweigt oder geradkettig sein. Vorzugsweise verwendet man als Mercaptocarbonsäure 2-Mercaptoessigsäure oder 3-Mercaptopropionsäure.

Die Veresterung des Polyvinylalkohols mit der Mercaptocarbonsäure kann unter den Bedingungen durchgeführt werden, welche man üblicherweise zur Veresterung von primären oder sekundären Alkoholen mit sterisch ungehinderten Mercaptocarbonsäuren anwendet.

Eine wenig aufwendige Veresterungsmethode ist ein Verfahren, bei dem der Polyvinylalkohol und die Mercaprocarbonsäure im molaren Verhältnis von etwa 1:1 bis 4:1 bezogen auf die Hydroxygruppen des Polyvinylalkohols in wässriger Lösung in Gegenwart starker Säuren - wie zum Beispiel Salzsäure, Schwefelsäure- oder stark saure Ionenaustauscher - wie zum Beispiel Amberlite(R)- IR-120- erhitzt. Nach erfolgter Umsetzung wird die Säure - beispielsweise durch Neutralisation und anschließende Dialyse oder durch Kationenaustauscher, wie Amberlite(R)- IR-4B, entfernt - beziehungsweise den stark sauren Ionenaustauscher abfiltriert. Das erhaltene Produkt kann als Lösung oder nach Einengen im Vakuum oder Gefriertrocknen als Feststoff weiter umgesetzt werden. Der Feststoff sollte etwa 0,4 bis 1,5 Milliäquivalente Mercaptogruppen pro g enthalten (Die Bestimmung kann nach Anal. Biochem. 94, 1979,75 erfolgen).

Die zum Zweck der Immobilisierung der Mikroorganismen folgende Vernetzung des mit der Mercaptocarbonsäure veresterten Polyvinylalkohols erfolgt vorzugsweise durch Luftsauerstoff.

Vorzugsweise wird der Mikroorganismus in einer eventuell gepufferten 1 bis 20 %igen wässrigen Lösung des mit der Mercaptocarbonsäure veresterten Polyvinylalkohols suspendiert,

zu einer dünnen Schicht ausgebreitet bei 0° C bis 20° C aufbewahrt. Nach erfolgter Vernetzung wird der Biokatalysator gewaschen, gewünschtenfalls gefriergetrocknet und als Folie oder in zerkleinerter Form verwendet. Wird der Biokatalysator in zerkleinerter Form verwendet, so ist es zweckmäßig ihn nach der Zerkleinerung naß zu sieben und die Siebfraktionen von 0,02 bis 2 mm, vorzugsweise solche von 0,05 bis 1,5 mm Korngröße zur Umsetzung von Steroiden zu verwenden.

Zur Durchführung des erfindungsgemäßen Verfahrens werden pro 100 ml einer 0,1 bis 5 gewichtsprozentigen Suspension des Biokatalysators in Wasser oder einer geeigneten Pufferlösung 0,03 bis 2 g des mikronisierten Steroids (die maximale Substratkonzentration ist abhängig von der Struktur des umzusetzenden Steroids) zugesetzt und man inkubiert die Reaktionsmischung bis zur Beendigung der Umsetzung. Oft ist es zweckmäßig das mikronisierte Steroid (Korngröße vorzugsweise 0,1 bis 50µ) zusätzlich noch zu emulgieren. Das kann geschehen, indem man es unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin(R), Tagat(R), Tween(R) und Span(R) beispielsmäßig genannt.

Die optimale Substratkonzentration, Substratzugabezeit und Fermentationsdauer ist von der Art der angewendeten Reaktionsbedingungen abhängig. Diese Größen müssen, wie dies bei mikrobiologischen Steroidumwandlungen allgemein erforderlich ist, im Einzelfall durch Vorversuche, wie sie dem Fachmann

geläufig sind, ermittelt werden.

Nach erfolgter Fermentation wird der Biokatalysator mechanisch wie zum Beispiel mittels eines Siebes abgetrennt, gewaschen und kann dann erneut zur Fermentation eingesetzt werden.

Die nachfolgenden Ausführungsbeispiele dienen zur näheren Erläuterung des erfindungsgemäßen Verfahrens.

Beispiel 1

a) Zwei 2 l Erlenmeyerkolben, von denen jeder 500 ml einer sterilisierten Nährlösung aus 0,5 % Corn steep, 0,1 % Hefeextrakt und 0,05 % Stärkezucker, eingestellt auf pH 7,0 enthält, wird mit einer Lyophilkultur von Arthrobacter simplex (ATCC 6946) beimpft und 48 Stunden bei 30° C auf einem Rotationsschüttler geschüttelt. Diese Vorkultur dient zur Beimpfung eines 50 l Glasfermenters, der mit 30 l eines sterilisierten Mediums aus 0,5 % Corn steep, 0,1 % Hefeextrakt und 0,05 % Stärkezucker, eingestellt auf pH 7,0, gefüllt ist. Nach der Beimpfung wird bei 29° C unter Belüftung (10 Liter/Min.), 0,7 atü Druck und Rühren (220 Umdrehungen pro Minute) 24 Stunden germiniert. Mit dieser Vorfermenter-Kultur wird nun ein Stahlfermenter beimpft, der 500 l eines bei 121° C und 1,1 atü sterilisierten Mediums aus 0,5 % Corn steep, 0,1 % Hefeextrakt und 0,05 % Stärkezucker, eingestellt auf pH 7,0, enthält und unter den Bedingungen des Vorfermenters ebenfalls 24 Stunden bis zur optimalen Zelldichte germiniert.

Anschließend wird die Kultur in einem Labor Separator LG 205 [Firma Westfalia Separator AG/Oelde (Westf.)] bei 10 000 Umdrehungen/Min. separiert. Die abgeschleuderte Zellmasse wird zur Entfernung anhaftender Bestandteile des Nährmediums mit destillierten Wasser gewaschen und erneut zentrifugiert.

Die auf diese Weise dargestellte feuchte Bakterienpaste (2,85 kg) wird in 2,85 l eines 0,05 molaren Tris-(hydroxymethyl)-methylamin-Puffers von pH 7,6 aufgeschlemmt, zur Zerteilung von Zellklumpen durch ein Metallgazefilter

gedrückt und in einem Universal-Labor-Zerstäubungstrockner [Firma Zahn & Co., Hameln, Deutschland] bei einer Verdampfungstemperatur von 80° C versprüht, wobei 600 ml Zellsuspension pro Stunde eingespeist werden.

Man erhält so 513 g Arthrobacter simplex Trockenpulver.

b) 11 g Polyvinylalkohol (Molgewicht ca. 72 000 Herstellerfirma Merck AG) wird in 125 ml 0,33 N wässriger Salzsäure bei 80° C gelöst. Dann setzt man unter Stickstoff langsam 8,8 g Mercaptopropionsäure zu, erhitzt noch 3 Stunden lang bei 90° C unter Rühren und rührt weitere 24 Stunden lang bei Raumtemperatur.

Dann neutralisiert man die Reaktionsmischung mit verdünnter Natronlauge dialysiert unter Stickstoff zwei Tage lang gegen Wasser verdünnt sie mit Wasser auf 250 ml und setzt ihr soviel Dinatriumhydrogenphosphat und Kaliumdihydrogenphosphat zu, daß die Konzentration an beiden Phosphaten je 25 millimolar ist. (Der Mercaptogruppengehalt des Polymeren liegt bei 0,13 m Äquiv./g)

c) 40 ml der gemäß b) hergestellten Polymeren-Lösung werden mit einer Suspension von 1,92 g Arthrobacter simplex Trockenpulver versetzt, verrührt und in eine Glasschale von 20 cm Durchmesser gegossen und 8 Tage lang bei 2° C aufbewahrt.

Der als Folie anfallende Biokatalysator wird 24 Stunden lang in Phosphatpuffer bei 30° C geschüttelt und dann lyophylisiert. Dann wird er zermahlen und die Teilchen mit einer Korngröße von 0,5 bis 1 mm ausgesiebt. Der so erhaltene Biokatalysator kann monatelang gelagert werden, ohne daß er in seiner Aktivität nachläßt.

d) Hydrocortison wird in einer Turbulenzmühle mikronisiert bis seine Teilchengröße überwiegend 1 bis 20 µ beträgt.

e) 36,2 mg mikronisiertes Hydrocortison werden in 50 ml Phosphat-Puffer (25 m mol Dinatriumhydrogenphosphat und 25 m mol Kaliumdihydrogenphosphat pro Liter) mit 100 mg lyophilisiertem Biokatalysator versetzt und bei 30° C mit 160 Umdrehungen pro Minute geschüttelt, bis zur vollständigen Umsetzung des Substrats (Dauer ca. 24 Stunden).

Nach beendeter Umsetzung wird der Biokatalysator von der Prednisolon-Suspension durch Sieben abgetrennt .In der gleichen Weise kann der Biokatalysator abermals mit mikronisierter Hydrocortison-Suspension umgesetzt werden. Selbst nach zehnmaliger Wiederholung hat der Biokatalysator noch seine volle Aktivität beibehalten.

Beispiel 2

Unter den Bedingungen des Beispiels 1 können folgende Steroide in der $\Delta^1$-Position dehydriert werden:

4-Östren-3,17-dion (hier entsteht durch Doppelbindungsisomerisierung Östron),
4-Androsten-3,17-dion,
17β-Hydroxy-4-androsten-3-on,
4-Pregnen-3,20-dion,
17α-Hexanoyloxy-4-pregnen-3,20-dion,
11β,21-Dihydroxy-4-pregnen-3,20-dion,
17α,21-Dihydroxy-4-pregnen-3,11,20-trion,
11β,21-Dihydroxy-6α-methyl-17α-propionyloxy-4-pregnen-3,20-dion,
6α-Fluor-11β,21-dihydroxy-16α-methyl-4-pregnen-3,20-dion,
21-Hydroxy-16α,17α-isopropylidendioxy-4,11-pregnadien-3,20-dion
21-Hydroxy-17α-propionyloxy-16β-methyl-4,11-pregnadien-3,20-dion und
9β,11β-Epoxy-21-hydroxy-16β-methyl-4-pregnen-3,20-dion.

Beispiel 3

a) 22 g Polyvinylalkohol (Molgewicht ca. 72 000 Herstellerfirma Merck AG) werden in 250 ml 0,33 N wässriger Salzsäure bei 80° C gelöst. Dann setzt man unter Stickstoff langsam 15,3 g Mercaptoessigsäure zu, erhitzt noch 3 Stunden lang bei 90° C unter Rühren und rührt weitere 24 Stunden lang bei Raumtemperatur.

Dann neutralisiert man die Reaktionsmischung mit verdünnter Natronlauge dialysiert sie zwei Tage lang gegen Wasser und lyophilisiert sie. (Der Thiolgruppengehalt des Polymeren liegt bei 0,12 m Äquiv./g.)

b) 10 ml einer 8 Gewichtprozentigen Lösung des gemäß b) hergestellten Polymeren in Phosphatpuffer (25 m mol Dinatriumhydrogenphosphat und 25 m mol Kaliumdihydrogenphosphat pro Liter) werden mit einer Suspension von 2,00 g Arthrobacter simplex - hergestellt nach Beispiel 1 a) - Trockenpulver in 30 ml des gleichen Phosphatpuffers versetzt, verrührt in 2 Glasschalen von je 20 cm Durchmesser gegossen und 8 Tage lang bei 2° C aufbewahrt.

Der als Folie anfallende Biokatalysator wird 24 Stunden lang in Phosphatpuffer bei 30° C gewaschen.

c) Hydrocortison wird in einer Turbulenzmühle mikronisiert bis seine Teilchengröße überwiegend 1 bis 20 μ beträgt.

d) 36,2 mg mikronisiertes Hydrocortison werden in 50 ml Phosphat-Puffer (25 m mol Dinatriumhydrogenphosphat und 25 m mol Kaliumdihydrogenphosphat pro Liter) mit 400 mg der feuchten Biokatalysatorfolie versetzt und bei 30° C mit 160 Umdrehungen pro Minute geschüttelt, bis das Substrat völlig umgesetzt ist (Dauer ca. 30 Stunden).

Nach beendeter Umsetzung wird der Biokatalysator aus der Prednisolon-Suspension herausgenommen. In der gleichen Weise kann der Biokatalysator abermals mit mikronisierter Hydrocortison-Suspension umgesetzt werden. Selbst nach fünfmaliger Wiederholung hat der Biokatalysator noch seine volle Aktivität beibehalten.

Beispiel 4

362 mg mikronisiertes Hydrocortison werden in 200 ml Phosphat-Puffer (25 m mol Dinatriumhydrogenphosphat und 25 m mol Kaliumdihydrogenphosphat pro Liter) suspendiert, mit 0,5 g lyophilisiertem Biokatalysator - hergestellt nach Beispiel 1- versetzt und bei 30° C mit 160 Umdrehungen pro Minute bis zur völligen Umsetzung (ca. 48 Stunden lang) geschüttelt.

Dann wird der Biokatalysator durch Sieben abgetrennt, mit Phosphatpuffer vom Sieb gespült, mit einer Suspension von 362 mg mikronisiertem Hydrocortison versetzt, das Gemisch mit Phosphatpuffer auf 200 ml aufgefüllt und wiederum bis zur völligen Umsetzung des Substrats bei 30° C mit 160 Umdrehungen pro Minute geschüttelt.

Man wiederholt diese Umsetzung noch 9 mal mit dem gleichen Biokatalysator - wobei der Biokatalysator nach den einzelnen Umsetzungen wochenlang in Phosphatpuffer bei +4° C aufbewahrt werden kann, ehe er zur nächsten Umsetzung verwendet wird.

Die erhaltenen Prednisolon-Suspensionen werden jeweils mit Chloroform extrahiert und die organischen Phasen im Vakuum eingeengt.

Die vereinigten Prednisolon-Rohprodukte werden aus Aceton umkristallisiert. Man erhält 3,28 g Prednisolon (= 91 % der Theorie) vom Schmelzpunkt 238-241° C.

Patentansprüche

1. Verfahren zur Herstellung von 3-Oxo-$\Delta^{1,4}$-steroiden aus in der 1,2-Position gesättigten 3-Oxo-$\Delta^{4}$-steroiden durch Fermentation mit einem Biokatalysator, in welchem Mikroorgnismen der Spezies Arthrobacter simplex oder Bacillus sphaericus in vernetztem, mit einer Mercaptocarbonsäure der allgemeinen Formel I

$$HOOCC_nH_{2n}SH \qquad (I),$$

worin n die Ziffern 1 bis 5 bedeuten, veresterten Polyvinylalkohol immobilisiert sind, dadurch gekennzeichnet, daß man den Biokatalysator einer wässrigen Suspension des mikronisierten 3-Oxo-$\Delta^{4}$-steroids zusetzt, die Reaktionsmischung bei einer Temperatur von 20 bis 40° C bis zur Umsetzung des Steroids inkubiert und danach den Biokatalysator mechanisch von der wässrigen Suspension des gebildeten 3-Oxo-$\Delta^{1,4}$-steroids abtrennt.

2. Verfahren zur Herstellung von 3-Oxo-$\Delta^{1,4}$-steroiden der allgemeinen Formel II

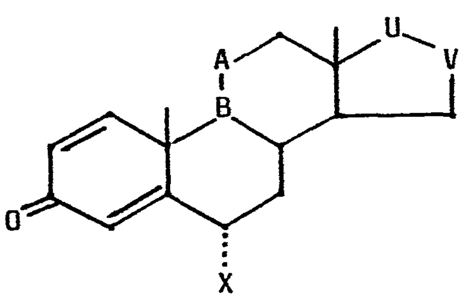

(II),

worin

X ein Wasserstoffatom, ein Chloratom, ein Fluoratom oder eine Methylgruppe bedeutet,

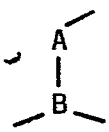 die Gruppierungen

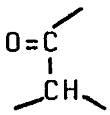 , 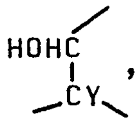 , 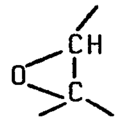 oder 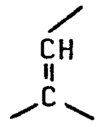

mit Y in der Bedeutung von Wasserstoff, Fluor oder Chlor und

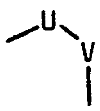 die Gruppierungen

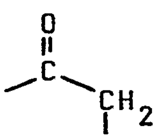 , 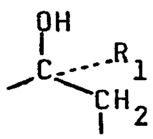 , 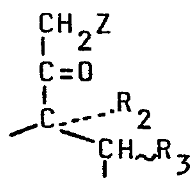 , 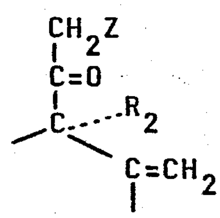 ,

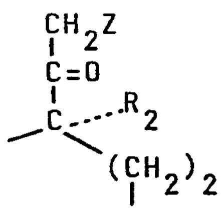 oder 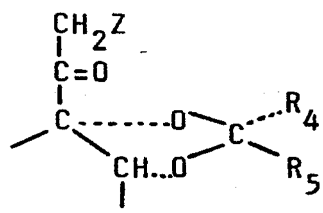

mit

Z in der Bedeutung eines Wasserstoffatoms, einer Hydroxygruppe eines Fluoratoms oder eines Chloratoms,

$R_1$ in der Bedeutung eines Wasserstoffatoms oder eines alicyclischen Kohlenwasserstoffrests mit 1 bis 4 Kohlenstoffatomen,

$R_2$ in der Bedeutung eines Wasserstoffatoms, einer Hydroxygruppe, einer gegebenenfalls durch ein Sauerstoffatom oder ein Schwefelatom unterbrochenen Alkylgruppe mit 1 bis 6 Kohlenstoffatomen, einer Alkanoyloxygruppe mit 1 bis 6 Kohlenstoffatomen oder einer Benzoyloxygruppe

$R_3$ in der Bedeutung eines Wasserstoffatoms, einer Hydroxygruppe oder einer Methylgruppe und

$R_4$ und $R_5$ in der Bedeutung einer Alkylgruppe mit 1 bis 4

0126877

Kohlenstoffatomen, darstellen, gemäß Anspruch 1, dadurch gekennzeichnet, daß man ein 3-Oxo-$\Delta^4$-steroid der allgemeinen Formel III

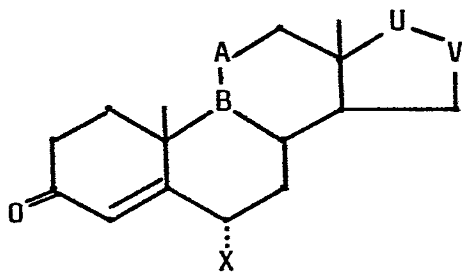

(III),

worin

X, 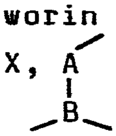 und 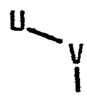 die obengenannte Bedeutung besitzen,

oder deren 21-Ester von Alkancarbonsäuren mit 1 bis 6 Kohlenstoffatomen fermentiert.

Europäisches Patentamt

# EUROPÄISCHER RECHERCHENBERICHT

0126877
Nummer der Anmeldung

EP 84 10 3034

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| X | DIE ANGEWANDTE MAKROMOLEKULARE CHEMIE, Band 104, 16. April 1982, Seiten 39-58, Hüthig & Wepf Verlag,Basel; CH; G. MANECKE et al.: "Immobilisierung von Corlynebakterium simplex in thioliertem Polyvinylalkohol zur mikrobiologischen Steroidumwandlung" * Insgesamt * --- | 1,2 | C 12 P 33/02<br>C 07 J 5/00 |
| A | BULLETIN OF THE ACADEMY OF SCIENCES OF THE USSR, Division of Chemical Science, (A translation of Izvestiya Akademii Nauk SSSR, Seriya Khimicheskaya), Band 25, Nr. 6, Juni 1976, Seiten 1303-1305, New York, USA; N.E. VOISHVILLO et al.: "Transformed steroids. Communication 81, Preparation of delta1,4-3 -ketosteroids employing immobilized bacterial cells" * Ingesamt * --- -/- | 1,2 | RECHERCHIERTE SACHGEBIETE (Int. Cl. 3)<br>C 12 P 33/00 |

Der vorliegende Recherchenbericht wurde für alle Patentansprüche erstellt.

| Recherchenort | Abschlußdatum der Recherche | Prüfer |
|---|---|---|
| DEN HAAG | 07-08-1984 | HENRY J.C. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82

Europäisches Patentamt

# EUROPÄISCHER RECHERCHENBERICHT

0126877

Nummer der Anmeldung

EP 84 10 3034

Seite 2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | CHEMICAL ABSTRACTS, Band 86, Nr. 25, 20. Juni 1977, Seite 430, Nr. 187642b, Columbus, Ohio, USA; P.O. LARSSON et al.: "Immobilization of steroid-transforming microorganisms in polyacrylamide" & METHODS ENZYMOL. 1976, 44(Immobilized Enzymes), 183-190 * Zusammenfassung * ----- | 1,2 | |
| | | | RECHERCHIERTE SACHGEBIETE (Int. Cl. 3) |

Der vorliegende Recherchenbericht wurde für alle Patentansprüche erstellt.

| Recherchenort | Abschlußdatum der Recherche | Prüfer |
|---|---|---|
| DEN HAAG | 07-08-1984 | HENRY J.C. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument